# EUROPÄISCHE PATENTSCHRIFT

(11) **EP 2 496 272 B1**
(45) Veröffentlichungstag und Bekanntmachung des Hinweises auf die Patenterteilung: **19.07.2017**
(21) Anmeldenummer: 10779242.6
(22) Anmeldetag: 05.11.2010
(51) Int. Cl.: A61L 9/12, A61L 9/03

(54) **VERFAHREN UND VORRICHTUNG ZUR ERZEUGUNG EINES DUFTLUFTSTROMS**
METHOD AND DEVICE FOR PRODUCING A FRAGRANCED AIR STREAM
PROCÉDÉ ET DISPOSITIF DE PRODUCTION D'UN COURANT D'AIR PARFUMÉ

(30) Priorität: 06.11.2009 DE 102009052267
(43) Veröffentlichungstag der Anmeldung: 12.09.2012
(73) Patentinhaber: Linde AG, 80331 München (DE)
(72) Erfinder: ORTNER, Georg, 50670 Köln (DE); KÄLLGREN, Olof, 82049 Pullach (DE)
(74) Vertreter: Grättinger Möhring von Poschinger Patentanwälte Partnerschaft
(86) Internationale Anmeldenummer: PCT/EP2010/006748
(87) Internationale Veröffentlichungsnummer: WO 2011/054529

(56) Entgegenhaltungen:
- EP-A1- 1 510 228
- DE-A1- 19 513 293
- GB-A- 2 122 903
- US-A- 6 136 277
- US-A1- 2004 003 812

## Beschreibung

Die Erfindung betrifft ein Verfahren und eine Vorrichtung zur Erzeugung eines Duftluftstroms gemäß den Oberbegriffen der unabhängigen Ansprüche 1 und 8.

Bekannte Vorrichtungen (US 6,136,277, EP 1 510 228 A1, US 3,410,488) besitzen innerhalb eines Gehäuses einen umlaufenden bandförmigen Duftstoffträger auf welchen gezielt Duftstoff aufgebracht wird. Neben dem Duftstoffträger mit Antriebsteilen ist jeweils auch eine Dosiervorrichtung im Gehäuseinneren angeordnet. Ein das Gehäuse durchströmender Frischluftstrom verwirbelt den auf dem Duftstoffträger in flüssiger Form aufgebrachten Duftstoff im Inneren des Gehäuses und tritt aus diesem als Duftluftstrom aus, wobei eine Teilmenge feinste Flüssigkeitspartikel des Duftstoffs vom Duftluftstrom erfasst wird. Im Gehäuseinneren kommt es dabei schon nach kurzer Betriebszeit zu einer unerwünschten Oberflächenbeschichtung mit dort verwirbeltem Duftstoff.

Bei nachfolgendem Duftstoffwechsel führen bereits kleinste Mengen an vom Luftstrom erfassten Flüssigkeitspartikeln eines anderen Duftstoffs zu verfälschten Dufteindrücken, wenn vorher keine gründliche Reinigung des gesamten Geräts zur Beseitigung aller Reste des bisher verwendeten Duftstoffes durchgeführt wird.

Zum besseren Verständnis der Zusammenhänge und Ursachen sei vorausgesetzt, dass bei der Beduftung von Räumen eingesetzte Duftstoffe in aller Regel komplexe Gemische aus ätherischen Ölen, wie Orangenöl, Pfefferminzöl, etc. und Einzelduftstoffen sind. Bei letzteren handelt es sich wahlweise um natürliche, naturidentische, halbsynthetische oder vollsynthetische Düfte, beispielweise aus Geraniol, Menthol, Cedramber, Galaxolide usw.

Insgesamt stehen dem Parfumeur als Fachmann für Duftstoffgemische ca. 3000 Duftstoffkomponenten zur Verfügung. Diese sind gekennzeichnet durch unterschiedliche Verdunstungstemperaturen und -geschwindigkeiten, Duftstoffstärken, Oxidationsbeständigkeiten, Dauer ihrer Wirkungen usw.

Mit dem Duftstoff in Berührung kommende Flächen, insbesondere im Bereich der Dosiervorrichtung oder von Strömungskanälen führen trotz verhältnismäßig kurzer Wartungsintervalle zu nachteiligen Beschichtungen, z.B. durch Verharzungen von Duftstoffrückständen oder durch Versottung mit flüssigen Restbestandteilen der anströmenden Duftluft. Häufig werden schon nach Stunden die frischen Aromen überdeckt bzw. entmischt und weichen eher abgestanden und ausgelaugt empfundenen Duftstoffresten. Zu große Wartungsintervalle führen sehr rasch zur Kontaminierung ganzer Lüftungssysteme, oft verursacht durch Verwendung von Sprays als Dosiervorrichtungen. Demgegenüber wird auch durch Überdosierung der in ein Lüftungssystem eingebrachten Duftstoffmengen keine Verbesserung erreicht. Die Folge ist vielmehr eine Überreizung der Sinneswahrnehmung bei Menschen, die sich im bedufteten Raum aufhalten, häufig verbunden mit Kopfschmerzen.

Es versteht sich von selbst, dass in kontaminierten Lüftungssystemen spontane Duftwechsel wirkungslos sind, da die abgelagerten Duftstofftröpfchen noch nach Stunden oder sogar Tagen an die anströmende Frischluft abgegeben werden, mit der unangenehmen Folge, dass bei einem Duftwechsel undefinierbare Duftmischungen entstehen.

Demgegenüber liegt der vorliegenden Erfindung die Aufgabe zugrunde, die Technologie der Luftbeduftung und des Duftwechsels derart zu verbessern, dass eine rasche Entmischung und Oxidation der Duftstoffe verhindert wird, dass eine Ablagerung von Duftstoffen auf der Geräteseite weitgehend unterbleibt und dass ein rascher Duftstoffwechsel ohne damit verbundene Wirkungsbeeinträchtigung möglich ist, wobei die Wartungsintervalle der Geräte deutlich verlängert werden. Außerdem soll die erfindungsgemäße Technologie eine weitgehende Automatisierung bei der Erzeugung und Verbreitung des Duftluftstroms und dessen genaue Dosierung nach vorgegebener Duftstärke ermöglichen.

Die vorstehende Aufgabe wird nach dem erfindungsgemäßen Verfahren gemäß dem Kennzeichen von Patentanspruch 1 gelöst.

Dabei ist sowohl beim Aufbringen des Duftstoffs auf den Duftstoffträger als auch bei dessen Durchlaufen des Verdunstungsschachtes von besonderer Bedeutung, dass der Duftstoff auf den fortbewegten bandförmigen Duftstoffträger ohne Benetzung der Umgebung z.B. in einer Dosiervorrichtung aufgebracht wird, vorteilhaft durch gezieltes Aufspritzen auf den Duftstoffträger vor dessen Eintreten in den Verdunstungsschacht und dass ferner im Verdunstungsschacht der benetzte Duftstoffträger möglichst mit keinem Wandteil des Verdunstungsschachtes in direkten Kontakt kommt. Damit wird gewährleistet, dass die im Verdunstungsschacht am Duftstoffträger vorbei streichende Frischluft den Duftstoff ausschließlich im gasförmigen Zustand aufnimmt, d.h. in Form der aus dem flüssig aufgebrachten Duftstoff durch Verdunsten austretenden Moleküle des Duftstoffgemisches. Auf diese Weise unterbleibt jeder Transport flüssiger Duftstofftröpfchen im Duftluftstrom und damit die Gefahr der geräteseitigen Kontaminierung.

Zur Förderung der Verdunstung im Verdunstungsschacht wird dieser vorteilhaft von erwärmter Frischluft durchströmt. Deren Temperatur sollte dabei deutlich über Raumtemperatur liegen, um sicherzustellen, dass alle Duftstoffanteile während des Duftstofftransports im Verdunstungsschacht verdunsten können. Die Verdunstung soll somit beschleunigt werden, wobei ein möglichst kurzzeitiges Aufwärmen anzustreben ist, um die Qualität der Duftstoffe zu erhalten.

Zu beachten ist dabei zweckmäßigerweise, dass der Duftstoffträger mit einstellbarer Geschwindigkeit durch den Verdunstungsschacht hindurch gezogen wird. Auf diese Weise gelingt es, in Abstimmung mit der Temperatur der angewärmten Frischluft ein optimales Verdunstungsergebnis bei kontrollierter Konzentration und gleichbleibender Qualität des Duftstoffes im Duftluftstrom zu erzielen.

Durch die weitere Ausgestaltung des erfindungsgemäßen Verfahrens, wonach die auf den Duftstoffträger je Längeneinheit aufgebrachte Duftstoffmenge zur Bestimmung der Duftstoffkonzentration feinregulierbar ist, wird eine weitgehend automatisch ablaufende Beduftung angestrebt, welche z.B. mittels geeigneter Programme durch elektronische Datenverarbeitung realisierbar ist. Diese ermöglichen nicht nur die Kontrolle der Beduftungsdauer bzw. die Erzielung einer gewünschten Duftstärke, sondern auch den Duftwechsel durch spontane Auswahl verschiedener Düfte, welche in einer Mehrzahl von alternativ zuschaltbaren Duftstoffbehältern bevorratet werden.

Diese Duftstoffbehälter werden vorteilhaft kühl gelagert, z.B. innerhalb eines Temperaturbereichs von 10-15°C. Zum Ausbringen des Duftstoffes aus dem Behälter steht dessen Inhalt zweckmäßig unter dem Druck eines inerten Gases wie Stickstoff (N2) oder Argon (Ar), wodurch eine schädliche Oxidation des Duftstoffes durch den Luftsauerstoff vermieden wird.

Eine erfindungsgemäße Vorrichtung, welche zur Durchführung des erfindungsgemäßen Verfahrens geeignet ist, ist dadurch gekennzeichnet, dass ein von Frischluft durchströmter Verdunstungsschacht vorgesehen ist, dass der Verdunstungsschacht eine Eintrittsöffnung für einen bandförmigen mit Duftstoff benetzten Duftstoffträger und eine Austrittsöffnung zu dessen Ausziehen aus dem Verdunstungsschacht nach Duftstoffabgabe in den Frischluftstrom aufweist und dass dem Verdunstungsschacht die Dosiervorrichtung vor- und eine Ausziehvorrichtung für den Duftstoffträger nachgeschaltet ist.

Der Duftstoffträger wird also mittels der Ausziehvorrichtung durch den Verdunstungsschacht hindurch gezogen, so dass er diesen durchquert während erwärmte Frischluft durch den Verdunstungsschacht hindurch strömt und sich dabei mit dem vom Duftstoffträger mitgeführten Duftstoff anreichert.

Dabei ist nach einer vorteilhaften Ausgestaltung vorgesehen, dass der Duftstoffträger aus einer Vorratstrommel heraus- und hintereinander durch die Dosiervorrichtung und den Verdunstungsschacht hindurch gezogen und zuletzt in eine Abfalltrommel hineingezogen wird.

Zu diesem Zweck umfasst die Ausziehvorrichtung bevorzugt zusammenwirkende Rotationskörper, zwischen denen der Duftstoffträger auf Zug befördert wird. Vorteilhaft kommen als Rotationskörper miteinander kämmende Zahnräder in Frage, derart, dass der Duftstoffträger schlupffrei durch den Verdunstungsschacht ziehbar ist. Zum Einbringen des verbrauchten Endes des Duftstoffträgers in die Abfalltrommel ist zweckmäßig eine weitere Zugvorrichtung innerhalb der Abfalltrommel vorgesehen, z.B. in Form einer rotierend angetriebenen Wickelrolle. Die Dosiervorrichtung ist erfindungsgemäß derart gestaltet, dass der Duftstoff in flüssiger Form verlustfrei auf den fortbewegten Duftstoffträger aufgespritzt werden kann. Hierzu umfasst die Dosiervorrichtung Düsen zum gezielten Aufspritzen feinster Duftstoffmengen auf den fortbewegten Duftstoffträger, letzterer beispielsweise ausgebildet als dochtartiger Faden, als Band, schmale Stoffbahn oder ggf. saugfähige Folie, welcher jeweils von einer entsprechenden Vorratsrolle aus der Vorratstrommel ausziehbar ist. Dabei versteht sich von selbst, dass der Duftstoff in Tröpfchenform auf der Oberfläche oder im Inneren des Duftstoffträgermaterials aufgenommen wird, um sich davon während des Durchlaufens des Verdunstungsschachtes gasförmig abzusondern, wobei die Duftstoffmoleküle von der den Verdunstungsschacht durchströmenden angewärmten Frischluft mitgerissen werden.

Ein möglichst rückstandfreies Aufbringen des Duftstoffes auf den Duftstoffträger ist besonders erwünscht; geeignet hierfür sind Techniken des Flüssigkeitstransports wie sie in ähnlicher Weise bei Tintenstrahldruckern zur Anwendung kommen.

Der aus dem Verdunstungsschacht austretende Duftluftstrom wird dann der Zuluft in einer Lüftungs- bzw. Klimaanlage zugemischt oder z.B. durch einen Lochboden einem daran angeschlossenen Raum zugeführt. Während des gesamten Transportweges des Duftstoffes, zunächst auf dem Duftstoffträger und danach in der Duftluft bzw. der Zuluft wird eine Tröpfchenausscheidung von Duftstoff vermieden, so dass eine Kontaminierung innerhalb der erfindungsgemäßen Beduftungsvorrichtung zuverlässig unterbleibt. Damit entfallen auch besondere Reinigungsmaßnahmen insbesondere auch im Zusammenhang mit einem Duftstoffwechsel. Dieser setzt lediglich voraus, dass die Dosiervorrichtung auf einen neuen Duftstoff umgeschaltet wird, was vorteilhaft erst dann geschieht, nachdem der Duftstoffträger, soweit er noch mit dem bisherigen Duftstoff benetzt ist, den Verdunstungsschacht vollständig durchlaufen hat.

Im Rahmen der Erfindung ist zweckmäßig, dass Dosiervorrichtung und Ausziehvorrichtung zur Steuerung der Dosiermenge elektronisch regelbar sind, ferner dass bei einer Mehrzahl von Duftstoffen der Duftwechsel mittels einer elektronischen Steuerung programmierbar ist. Eine Voraussetzung hierfür ist, dass eine Mehrzahl von Duftstoffbehältern mit unterschiedlichen Duftstoffen sequenziell ansteuerbar sind.

Mittels eines entsprechenden Computerprogramms besteht sodann die Möglichkeit, die erfindungsgemäße Vorrichtung vollautomatisch zu steuern, z.B. mit wechselnden Düften entsprechend einer vorgegebenen Bildfolge oder dem Ablauf eines Films, beispielsweise von Filmsequenzen zu Werbezwecken. Daran angepasst besteht die Möglichkeit, wechselnde Duftstoffe z.B. in der Zuluft eines Lüftungssystems anzubieten, d.h. mit steuerbarer Duftstoffkonzentration unterschiedliche Duftstoffqualitäten, erkennbar nach Blüten, Früchten, Hölzern, Gewürzen usw. zu liefern. Wenn Konsumenten beispielsweise einen anregenden Citrusduft nach einer längeren Einwirkungsdauer durch Gewöhnung nicht mehr wahrnehmen, so kann dem durch abwechselnde Zufuhr beruhigender Duftstoffe, z.B. charakterisiert nach Kräutern, Lindenblüten, Verbena oder anderer Aromastoffe wie z.B. Orange oder Grapefruit abgeholfen werden.

Im Rahmen der Erfindung kann neben der Beduftung von Räumen auch eine Beduftung anderer Objekte mit Hohlräumen wie beispielsweise Möbeln - Tische, Sessel, Schränke, Betten, Kommoden - verwirklicht werden.

Ferner kann die beduftete Frischluft auch direkt, d.h. ohne den Umweg über eine Klimaanlage einem zu beduftenden offenen oder geschlossenen Raum, wie z.B. einem Stadion oder einer Stadtlandschaft zugeführt werden. Schließlich kommt statt eines quer zur Strömungsrichtung geschlossenen Verdunstungsschachts auch ein quer zur Strömungsrichtung einseitig offener Verdunstungsschacht, z.B. in Art eines Bodenkanals, Orchestergrabens oder sogar eines natürlichen Taleinschnitts mit einer Windströmung in Frage.

Vorteilhaft dient Frischluft als Strömungsmedium für den im Verdunstungsschacht zugemischten Duftstoff; geeignet sind aber auch andere gasförmige Strömungsmedien, z.B. in Form elementarer Gase wie Stickstoff oder anderer inerter Gase.

Im Folgenden wird eine Ausführungsform der Erfindung anhand der Zeichnungen erläutert. Es zeigt:
- Fig. 1: eine Beduftungsvorrichtung in der Seitenansicht und
- Fig. 2: die Beduftungsvorrichtung gemäß Figur 1 in der Draufsicht.

Im Zentrum der Beduftungsvorrichtung befindet sich ein Verdunstungsschacht 1, welcher mit seinem unteren Ende an einen Frischluftkanal 2 angeschlossen ist. Die gemäß Pfeil F zuströmende Frischluft durchläuft eine Heizung 3, welche einen Abschnitt des Frischluftkanals 2 umgibt und die darin strömende Frischluft deutlich über Raumtemperatur aufwärmt. Zur Förderung der Verdunstung im Verdunstungsschacht 1 beträgt die Temperatur der in den Verdunstungsschacht 1 eintretenden Frischluft zwischen 40 und 80°C, wobei in Ausnahmefällen sogar noch höhere Temperaturen eingestellt werden.

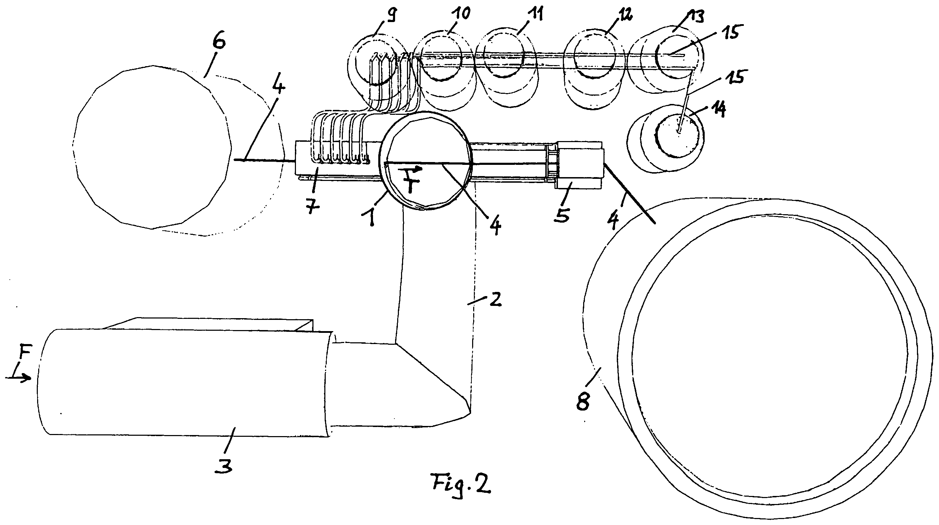

Ein dochtartiger Faden 4 dient als Duftstoffträger. Er durchquert, wie in Fig. 2 dargestellt den Verdunstungsschacht 1 in Richtung des Pfeils T. Der noch unbenetzte Duftstoffträger 4 wird mittels einer Ausziehvorrichtung 5, welche dem Verdunstungsschacht 1 nachgeschaltet ist, aus einer Vorratstrommel 6 herausgezogen, durchläuft eine Dosiervorrichtung 7, durchquert danach den Verdunstungsschacht 1 und gelangt nach der Ausziehvorrichtung 5 mittels einer nicht dargestellten Zugvorrichtung in das Innere einer Abfalltrommel 8.

An die Dosiervorrichtung 7 sind insgesamt sechs Duftstoffbehälter 9 bis 14 für unterschiedliche Duftstoffe angeschlossen, welche jeweils über Druckleitungen 15 mit nicht näher dargestellten Düsen innerhalb der Dosiervorrichtung 7 verbunden sind.

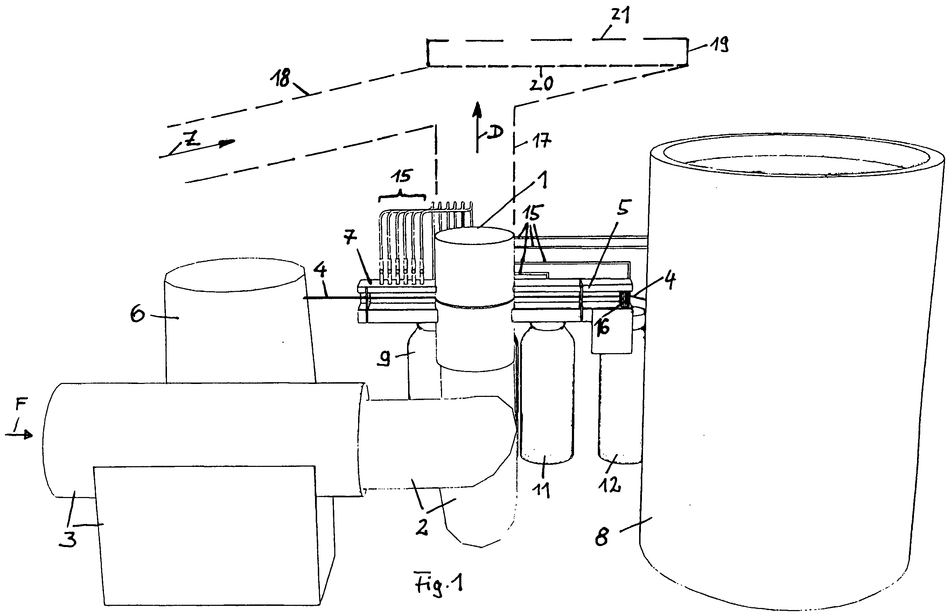

Gemäß Fig. 1 erkennt man den Duftstoffträger 4 an seinem Fadenverlauf durch die Dosiervorrichtung 7 bis zum Eintritt in den Verdunstungsschacht 1 und nach dem Austritt aus dem Verdunstungsschacht 1 weiter durch die Ausziehvorrichtung 5 und von dort weiter in die Abfalltrommel 8. Im Inneren der Ausziehvorrichtung 5 ist zur Gewährleistung eines schlupffreien Transports des bandförmigen Duftstoffträgers 4 ein Zahnrad 16 eines Zahnradpaars erkennbar, welches dem gleichförmigen Transport des Duftstoffträgers in Pfeilrichtung T dient.

Irgendwelche Steuerungsgeräte sind der Einfachheit halber auf der Zeichnung weggelassen. Man erkennt jedoch in strichlierter Ausführung die durch einen Beiluftkanal 17 aus dem Verdunstungsschacht 1 austretende Duftluft gemäß Pfeil D. Die Duftluft strömt in einen Zuluftkanal 18 einer Belüftungs- und Klimaanlage ein, wo sich die gemäß Pfeil Z zuströmende klimatisierte Zuluft mit der Duftluft aus dem Beiluftkanal 17 vermischt. Der Zuluftkanal 18 ist an einen Siebboden 19 angeschlossen, über welchen die beduftete Zuluft in den darüber befindlichen Raum gelangt. Der Siebboden 19 umfasst zwei Lochbleche, nämlich ein unteres Lochblech 20 mit verhältnismäßig kleinen Löchern und ein darüber angeordnetes oberes Lochblech 21 mit etwas größeren Austrittslöchern, durch welche die beduftete Zuluft in den Raum strömt. Das untere Lochblech 20 mit der kleineren Lochung bewirkt einen geringfügigen Rückstau der Zuluft, so dass damit eine gleichmäßige Strömungsverteilung erzielbar ist. Diese Anordnung erlaubt besonders rasche Duftstoffwechsel. Der Duftluftstrom lässt sich als bypass auch sehr einfach an eine bestehende Klimaanlage anschließen.

Dadurch, dass der bandförmige Duftstoffträger 4 praktisch berührungsfrei zwischen Vorratstrommel 6 und Abfalltrommel 8 gefördert wird, gelingt es, die Beduftungsvorrichtung ohne Kontaminierung derselben durch Duftstofftröpfchen zu betreiben. Erst dadurch ergibt sich ein problemloser Duftstoffwechsel, wobei beliebig viele Duftstoffbehälter anschließbar sind. Wichtig ist dabei, dass die Düsen am Ende der Druckleitungen 15 innerhalb der Dosiervorrichtung 7 derart ausgebildet sind, dass daraus Duftstofftröpfchen gezielt austreten können, so dass diese genau auf den bewegten Duftstoffträger auftreffen. Man wird daher den bandförmigen Duftstoffträger 4 derart dimensionieren und in seiner Saugfähigkeit derart auswählen, dass er in der Lage ist, den flüssigen Duftstoff ohne Zurückspritzen vollständig aufzunehmen, zugleich aber in der Lage ist, während der Durchquerung des Verdunstungsschachtes 1 die Verdunstung des Duftstoffes und dessen Übertritt in gasförmiger Form in den erwärmten Frischluftstrom zuzulassen. Das im Abfallbehälter aufgewickelte gebrauchte Ende des Duftstoffträgers 4 wird in gegebenen Intervallen als Abfall entsorgt. Aus der Vorratstrommel 6 wird stets nur reines Trägermaterial herausgezogen, welches nur für eine einmalige Verwendung vorgesehen ist.

## Patentansprüche

1. Verfahren zur Erzeugung eines Duftluftstroms, zur Beduftung von Räumen oder Objekten,
wobei Duftstoff gezielt auf einen bandförmigen Duftstoffträger (4) aufgebracht wird, der in einem Frischluftstrom fortbewegt wird,
**dadurch gekennzeichnet,**
**dass** der Duftstoff auf den laufenden Duftstoffträger (4) vor dessen Eintreten in einen Verdunstungsschacht (1) aufgebracht wird,
**dass** durch den Verdunstungsschacht (1) Frischluft hindurch geschickt wird, sodass der Duftstoff auf dem Duftstoffträger (4) während der Durchquerung des Verdunstungsschachts (1) verdunstet und
**dass** der Duftstoffträger (4) nach seinem Austreten aus dem Verdunstungsschacht (1) als Abfall beseitigt wird.

2. Verfahren nach Anspruch 1, **dadurch gekennzeichnet,**
**dass** die Frischluft auf eine Temperatur zwischen 40 und 80 °C erwärmt wird.

3. Verfahren nach Anspruch 1, **dadurch gekennzeichnet,**
**dass** der Duftstoffträger (4) mit einstellbarer Geschwindigkeit durch den Verdunstungsschacht (1) hindurchgezogen wird.

4. Verfahren nach Anspruch 1, **dadurch gekennzeichnet,**
**dass** die auf den Duftstoffträger (4) je Längeneinheit aufgebrachte Duftstoffmenge zur Bestimmung der Duftstoffkonzentration fein regulierbar ist.

5. Verfahren nach Anspruch 1, **dadurch gekennzeichnet,**
**dass** eine Mehrzahl verschiedener Duftstoffe in alternativ zuschaltbaren Duftstoffbehältern (9-14) bevorratet werden.

6. Verfahren nach Anspruch 5, **dadurch gekennzeichnet,**
**dass** die Duftstoffbehälter (9-14) innerhalb eines Temperaturbereichs von 10 - 15 °C gekühlt gelagert werden.

7. Verfahren nach Anspruch 5,
**dadurch gekennzeichnet,**
**dass** das Ausbringen des Duftstoffs aus den Duftstoffbehältern (9-14) unter dem Druck eines inerten Gases erfolgt.

8. Vorrichtung zur Erzeugung eines Duftluftstromes zur Beduftung von Räumen oder Objekten mit einer Dosiervorrichtung (7) zum Einmischen eines oder mehrerer Duftstoffe aus separaten Duftstoffbehältern (9-14),
**dadurch gekennzeichnet,**
**dass** ein von Frischluft durchströmter Verdunstungsschacht (1) vorgesehen ist,
**dass** der Verdunstungsschacht (1) eine Eintrittsöffnung für einen bandförmigen, mit Duftstoff benetzten Duftstoffträger (4) und eine Austrittsöffnung zu dessen Ausziehen aus dem Verdunstungsschacht (1) nach Duftstoffabgabe in den Frischluftstrom aufweist und
**dass** dem Verdunstungsschacht (1) die Dosiervorrichtung (7) vor- und eine Ausziehvorrichtung (5) für den Duftstoffträger (4) nachgeschaltet ist.

9. Vorrichtung nach Anspruch 8, **dadurch gekennzeichnet,**
**dass** der Duftstoffträger (4) mittels der Ausziehvorrichtung (5) aus einer der Dosiervorrichtung (7) vorgeschalteten Vorratstrommel (6) heraus- und zuletzt in eine dem Verdunstungsschacht (1) nachgeschaltete Abfalltrommel (8) hineingezogen wird.

10. Vorrichtung nach Anspruch 8, **dadurch gekennzeichnet,**
**dass** die Ausziehvorrichtung (5) zusammenwirkende Rotationskörper, zwischen denen der Duftstoffträger (4) auf Zug befördert wird, umfasst.

11. Vorrichtung nach Anspruch 10, **dadurch gekennzeichnet,**
**dass** die Rotationskörper miteinander kämmende Zahnräder (16) sind, derart, dass der Duftstoffträger (4) schlupffrei durch den Verdunstungsschacht (1) ziehbar ist.

12. Vorrichtung nach Anspruch 8, **dadurch gekennzeichnet,**
**dass** die Dosiervorrichtung (7) Düsen zum gezielten Aufspritzen von Duftstoff auf den fortbewegten Duftstoffträger (4) umfasst.

13. Vorrichtung nach Anspruch 8, **dadurch gekennzeichnet,**
**dass** der Duftluftstrom dem Zuluftkanal (18) einer Lüftungs- bzw. Klimaanlage zugemischt wird.

14. Vorrichtung nach Anspruch 8, **dadurch gekennzeichnet,**
**dass** der Duftluftstrom durch einen Lochblechboden (19) in einen Raum eingeleitet wird.

15. Vorrichtung nach Anspruch 8, **dadurch gekennzeichnet,**
**dass** Dosiervorrichtung (7) und Ausziehvorrichtung (5) zur Steuerung der Dosiermenge elektronisch regelbar sind.

16. Vorrichtung nach Anspruch 8, **dadurch gekennzeichnet,**
**dass** bei einer Mehrzahl von Duftstoffen der Duftwechsel mittels einer elektronischen Steuerung programmierbar ist.

17. Vorrichtung nach Anspruch 8, **dadurch gekennzeichnet,**
**dass** eine Mehrzahl von Duftstoffbehältern (9-14) mit unterschiedlichen Duftstoffen sequentiell ansteuerbar sind.

## Claims

1. A method for producing a fragranced air stream for fragrancing rooms, objects or the like, comprising: purposeful applying a fragrance on a strip-like fragrance carrier (4), which is advanced in a fresh air stream, and
wherein the fragrance is applied onto the moving fragrance carrier (4) before it enters an evaporation shaft (1), and wherein fresh air is passed through the evaporation shaft (1), and in that fragrance on the fragrance carrier (4) evaporates during its passage through the evaporation shaft (1), and in that the fragrance carrier (4) is eliminated as waste after it has exited the evaporation shaft (1).

2. A method according to claim 1, wherein the fresh air is heated to a temperature between 40° and 80°C.

3. A method according to claim 1, wherein the fragrance carrier (4) is pulled through the evaporation shaft (1) with adjustable speed.

4. A method according to claim 1, wherein the amount of fragrance applied on the fragrance carrier (4) per unit length can be finely regulated to determine the fragrance concentration.

5. A method according to claim 1, wherein a plurality of different fragrances are stored in alternatively connectable fragrance containers (9-14).

6. A method according to claim 5, wherein the fragrance containers (9-14) are stored within a temperature range of 10 - 15°C under cool conditions.

7. A method according to claim 5, wherein the fragrance is spreaded from the fragrance containers (9-14) with an inert gas under pressure.

8. A device for producing a fragranced air stream for fragrancing rooms or objects, comprising:
a dosing device (7) for admixing one or more fragrances from separate fragrance containers (9-14); and
an evaporation shaft (1) through which fresh air flows;
wherein the evaporation shaft (1) has an inlet aperture for a strip-like fragrance carrier (4) wetted with fragrance and an outlet aperture for extracting the strip-like fragrance carrier (4) from the evaporation shaft (1) after release of fragrance into the fresh air stream; and wherein the dosing device (7) is connected upstream and an extraction device (5) for the fragrance carrier (4) is connected downstream from the evaporation shaft (1) .

9. A device according to claim 8, wherein the fragrance carrier (4) is pulled by means of the extraction device (5) out of a supply drum (6) which is prepositioned to the closing device (7) and pulled successively through the dosing device (7) and the evaporation shaft (1) and ultimately pulled into a waste drum (8) .

10. A device according to claim 8, wherein the extraction device (5) comprises interacting rotating members, between which the fragrance carrier (4) is conveyed by traction.

11. A device according to claim 10, wherein the rotating members are toothed gears (16) meshing with one another in such a way that the fragrance carrier (4) can be pulled without slipping through the evaporation shaft (1).

12. A device according to claim 8, wherein the dosing device (7) comprises nozzles for purposeful spraying of fragrance onto the moving fragrance carrier (4).

13. A device according to claim 8, wherein the fragranced air stream is mixed with the supply air of a ventilation or air-conditioning system.

14. A device according to claim 8, wherein the fragranced air stream is passed through a perforated plate (19) into a room.

15. A device according to claim 8, wherein the dosing device (7) and extraction device (5) are electronically regulable for control of the dosing concentration.

16. A device according to claim 8, wherein, in the case of a plurality of fragrances, the change of fragrance is programmable by means of an electronic controller.

17. A device according to claim 8, wherein a plurality of fragrance containers (9-14) with different fragrances can be activated sequentially.

## Revendications

1. Procédé de production d'un courant d'air parfumé pour parfumer des espaces ou des objets,
un parfum étant appliqué de manière précise sur un support de parfum (4) en forme de bande, qui est déplacé dans un courant d'air frais, **caractérisé en ce que** le parfum est appliqué au support de parfum (4) en cours avant l'entrée de celui-ci dans un puits de vaporisation (1),
**en ce que** de l'air frais est envoyé à travers le puits de vaporisation (1) de telle manière que le parfum est vaporisé sur le support de parfum (4) pendant la traversée du puits de vaporisation (1) et
**en ce que** le support de parfum (4) est éliminé en tant que déchet après sa sortie du puits de vaporisation (1) .

2. Procédé selon la revendication 1, **caractérisé en ce que** l'air frais est réchauffé à une température se situant entre 40 et 80°.

3. Procédé selon la revendication 1, **caractérisé en ce que** le support de parfum (4) est tiré à travers le puits de vaporisation (1) à une vitesse réglable.

4. Procédé selon la revendication 1, **caractérisé en ce que** la quantité de parfum appliquée au support de parfum (4) par unité de longueur peut être régulée de manière précise pour déterminer la concentration de parfum.

5. Procédé selon la revendication 1, **caractérisé en ce qu'**une pluralité de parfums différents est en réserve dans des réservoirs de parfum (9-14) pouvant être alternativement mis en service.

6. Procédé selon la revendication 5, **caractérisé en ce que** les réservoirs de parfum (9-14) sont entreposés réfrigérés dans une plage de températures de 10 - 15 °C.

7. Procédé selon la revendication 5, **caractérisé en ce que** l'extraction du parfum des réservoirs de parfum (9-14) a lieu sous la pression d'un gaz inerte.

8. Dispositif pour la production d'un courant d'air parfumé pour parfumer des espaces ou des objets avec un dispositif de dosage (7) pour incorporer par mélange un ou plusieurs parfums à partir de réservoirs de parfum séparés (9-14), **caractérisé en ce qu'**un puits de vaporisation (1) traversé par de l'air frais est prévu,
**en ce que** le puits de vaporisation (1) comporte une ouverture d'entrée pour un support de parfum (4) en forme de bande, humecté avec du parfum et une ouverture de sortie pour l'extraction de celui-ci du puits de vaporisation (1) après dégagement du parfum dans le courant d'air frais et
**en ce que** le dispositif de dosage (7) est raccordé en amont et un dispositif d'extraction (5) en aval au puits de vaporisation (1) pour le support de parfum (4) .

9. Dispositif selon la revendication 8, **caractérisé en ce que** le support de parfum (4) est tiré hors d'un tambour de réserve (6) raccordé en amont au dispositif de dosage (7) au moyen du dispositif d'extraction (5) et finalement tiré à l'intérieur dans un tambour de déchets (8) raccordé en aval au puits de vaporisation (1) .

10. Dispositif selon la revendication 8, **caractérisé en ce que** le dispositif d'extraction (5) comprend des corps en rotation coopérant entre lesquels le support de parfum (4) est transporté par traction.

11. Dispositif selon la revendication 10, **caractérisé en ce que** les corps en rotation sont des engrenages (16) s'engrenant entre eux de telle manière que le support de parfum (4) peut être tiré sans glissement à travers le puits de vaporisation (1).

12. Dispositif selon la revendication 8, **caractérisé en ce que** le dispositif de dosage (7) comprend des buses pour pulvériser de façon précise du parfum sur le support de parfum (4) déplacé.

13. Procédé selon la revendication 8, **caractérisé en ce que** le courant d'air parfumé est ajouté au conduit d'arrivée d'air (18) d'une installation de ventilation ou de climatisation.

14. Dispositif selon la revendication 8, **caractérisé en ce que** le courant d'air parfumé est introduit dans un espace à travers un fond en tôle perforée (19).

15. Dispositif selon la revendication 8, **caractérisé en ce que** le dispositif de dosage (7) et le dispositif d'extraction (5) peuvent être réglés électroniquement pour commander la quantité de dosage.

16. Dispositif selon la revendication 8, **caractérisé en ce que** le changement de parfum peut être programmé au moyen d'une commande électronique pour une pluralité de parfums.

17. Dispositif selon la revendication 8, **caractérisé en ce qu'**une pluralité de réservoirs de parfum (9-14) peut être pilotée de manière séquentielle avec des parfums différents.
